# EUROPÄISCHE PATENTSCHRIFT

(11) **EP 0 697 854 B1**
(45) Veröffentlichungstag und Bekanntmachung des Hinweises auf die Patenterteilung: **05.03.1997**
(21) Anmeldenummer: 94917598.8
(22) Anmeldetag: 09.05.1994
(51) Int. Cl.: A61K 7/13

(54) **VERWENDUNG VON HYDROXYNAPHTHALINEN IN FÄRBEMITTELN**
USE OF HYDROXYNAPHTHALENES IN COLOURING AGENTS
UTILISATION D'HYDROXYNAPHTALENES DANS DES COLORANTS

(30) Priorität: 18.05.1993 DE 4316602
(43) Veröffentlichungstag der Anmeldung: 28.02.1996
(73) Patentinhaber: Henkel Kommanditgesellschaft auf Aktien, 40191 Düsseldorf (DE)
(72) Erfinder: ROSE, David, D-40723 Hilden (DE); Lieske, Edgar Deceased, (DE)
(86) Internationale Anmeldenummer: EP9401483
(87) Internationale Veröffentlichungsnummer: WO9426241

(56) Entgegenhaltungen:
- EP-A- 0 166 100
- EP-A- 0 345 728
- US-A- 4 003 699
- DATABASE WPI Week 9252, Derwent Publications Ltd., London, GB; AN 92-427913 & JP,A,4 323 274 (KENSINTON KK) 12. November 1992

## Beschreibung

Die Erfindung betrifft die Verwendung von bestimmten Hydroxynaphthalinen zum Färben von Keratinfasern, insbesondere menschlichen Haaren. Die Hydroxynaphthaline eignen sich besonders als Kupplerkomponenten gemeinsam mit üblichen Entwicklerkomponenten.

Oxidationsfärbemittel enthalten üblicherweise Oxidationsfarbstoffvorprodukte in einem wäßrigen Träger; als Oxidationsfarbstoffvorprodukte werden Entwicklerkomponenten und Kupplerkomponenten eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Aus der europäischen Offenlegungsschrift EP 166100 A1 ist die Verwendung von 4-Methoxy-1-naphthol als Kupplerkompnente in Oxidationshaarfärbemitteln bekannt. Die Verwendung von Alkoxynaphtholen zum Haarefärben, in denen Alkoxy- und Hydroxygruppe nicht an den selben 6-Ring des Naphthalingerüsts, sondern an unterschiedliche 6-Ringe gebunden sind, ist bislang unbekannt.

Es wurde nun gefunden, daß Naphthaline, die eine Hydroxygruppe und am anderen 6-Ring des Naphthalingerüsts einen unten näher bezeichneten Substituenten tragen, die an Färbemittelvorprodukte bzw. Färbemittel zu stellenden Anforderungen (hohe Intensität der Ausfärbung, Lichtechtheit, Waschechtheit, Reibechtheit) in besonderem Maße erfüllen.

Gegenstand der Erfindung ist die Verwendung von Hydroxynaphthalinen der allgemeinen Formel 1 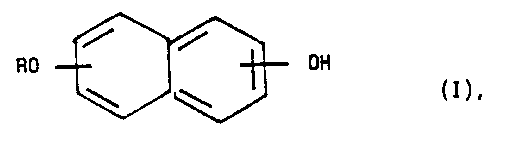 wobei R für eine Cᵢ-C₄-Alkyl-, eine C₂-C₄-Hydroxyalkyl-, eine Phenyl- oder eine Benzylgruppe steht, zum Färben von Keratinfasern, insbesondere menschlichen Haaren. Unter Keratinfasern sind Haare, Pelze, Wolle oder Federn zu verstehen.

Besonders eignen sich die Hydroxynaphthaline der Formel I, in denen R für eine C₁-C₄-Alkylgruppe steht. Insbesondere eignen sich 1-Hydroxy-6-methoxynaphthalin, 1-Hydroxy-7-methoxynaphthalin, 2-Hydroxy-6-methoxynaphtha- lin und 2-Hydroxy-7-methoxynaphthalin. Ganz besonders eignen sich die Hydroxynaphthaline der Formel I zum Färben von Keratinfasern, wenn sie gemeinsam mit üblichen Entwicklerkomponenten eingesetzt werden. Ein weiterer Patentgegenstand ist deshalb die Verwendung von Hydroxynaphtalinen der Fomel I als Kupplerkomponenten in Oxidationsfärbemitteln gemeinsam mit üblichen Entwicklerkomponenten zum Färben von Keratinfasern.

Übliche Entwiclderkomponenten sind z.B. primäre aromatische Amine mit einer weiteren in para- oder ortho-Position befindlichen freien oder substituierten Hydroxy- oder Aminogruppe, Diaminopyridin-Derivate, heterocyclische Hydrazone, 4-Aminopyrazolon-Derivate, Tetraaminopyrimidine, 2,4,5-Triamino-6-hydroxy-pyrimidine, 5,6-Diamino-2,4-dihydroxypyrimidine und deren Derivate. Speziell eignen sich p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanot, 1-Phenyl-3-carboxyamido-4-amino-pyrazolon-5, 4-Amino-3-methylphenol.

Die Hydroxynaphthaline der Formel I liefern mit den üblichen Entwicklerkomponenten ein breites Spektrum anwendungstechnisch interessanter Oxidationsfarben im Bereich roter bis blauer Nuancen, die sich wegen ihrer Brillanz und guten Echtheitseigenschaften insbesondere zum Färben von menschlichen Haaren eignen.

Ein weiterer Patentgegenstand sind deshalb Oxidationsfärbemittel zum Färben von menschlichen Haaren enthaltend Oxidationsfarbstoffvorprodukte in einem wasserhaltigen Träger, wobei die Hydroxynaphthaline der Formel I als Kuppler in einer Menge von 0,05 bis 20 mMol sowie übliche Entwicklerkomponenten in einer Menge von 0,05 bis 20 mMol und gegebenenfalls übliche direktziehende Farbstoffe in einer Menge von 0,05 bis 20 mMol, jeweils pro 100 g des Färbemittels, enthalten sind.

Übliche direktziehende Farbstoffe sind z.B. Nitrophenylendiamine, Nitroaminophenole, Anthrachinonfarbstoffe oder Indophenole. Die erfindungsgemäßen Haarfärbemittel können neben den Hydroxynaphthalinen der Formel 1 auch andere bekannte Kupplerkomponenten enthalten, die zur Modifizierung der Farbnuancen und zur Erzeugung natürlicher Farbtöne erforderlich sind. Solche üblichen Kupplerverbindungen sind z.B. meta-Phenylendiaminderivate, Naphthole, Resorcinderivate und Pyrazolone. Zur weiteren Modifizierung der Farbnuancen können gegebenenfalls auch mehrere bekannte Entwicklerkomponenten und direktziehende Farbstoffe eingesetzt werden.

Es ist auch nicht erforderlich, daß die Hydroxynaphthaline der Formel I einheitliche Verbindungen sind. Vielmehr können auch Gemische verschiedener Verbindungen der Formel I verwendet werden.

In den erfindungsgemäßen Haarfärbemitteln werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können. Zur Herstellung der erfindungsgemäßen Färbemittel werden die Oxidationsfarbstoffvorprodukte in einen geeigneten wasserhaltigen Träger eingearbeitet. Solche Träger sind z.B. Cremes, Emulsionen, Gele oder auch tensidhaltige schäumende Lösungen, z. B. Shampoos, Schaumaerosole oder andere Zubereitungen, die für die Anwendung auf dem Haar geeignet sind.

Der wasserhaltige Träger enthält üblicherweise Netz- und Emulgiermittel wie anionische, nichtionische oder ampholytische Tenside, z.B. Fettalkoholsulfate, Alkansulfonate, a-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z. B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Die Bestandteile des wasserhaltigen Trägers werden zur Herstellung der erfindungsgemäßen Haarfärbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht ist. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo verwendet wurde.

Die nachfolgenden Beispiele sollen den Erfindungsgegenstand näher erläutern, ohne ihn jedoch hierauf zu beschränken.

### Beispiel

Es wurden erfindungsgemäße Färbemittel in Form einer Haarfärbecreme-Emulsion der folgenden Zusammensetzung hergestellt:

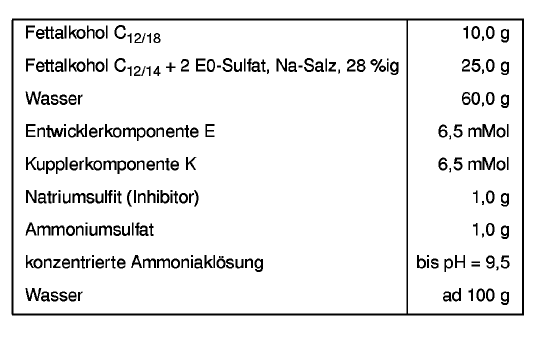

Als Hydroxynaphthaline der Formel I (Kupplerkomponenten K) wurden folgende Verbindungen eingsetzt:
K 1 : 2-Hydroxy-7-methoxynaphthalin (Synthese beschrieben in "Synlett (1991), 405")
K 2 : 1-Hydroxy-6-methoxynaphthalin (Synthese beschrieben in "Tetrahedron (1963), 19, 1932")
K 3 : 1-Hydroxy-7-methoxynaphthalin (Synthese beschrieben in "Tetrahedron (1963), 19, 1932")

Als Entwicklerkomponenten E wurden folgende Verbindungen eingesetzt:
E1: p-Toluylendiamin
E2: 2,4,5,6-Tetraaminapyrimidin
E3: p-Aminophenol
E4: N,N-Bis-(2-hydroxyethyl)-p-phenylendiamin
E5: 2-(2,5-Diaminophenyl)-ethanol
E6: 2-(2,5-Diaminophenoxy)-ethanol
E7: 1-Phenyl-3-carboxyamido-4-amino-pyrazolon-5
E8: 4-Amino-3-methylphenol

Die Bestandteile wurden der Reihe nach miteinander vermischt. Nach Zugabe der Oxidationsfarbstoffvorprodukte und des Inhibitors wurde zunächst mit konzentrierter Ammoniak lösung der pH-Wert der Emulsion auf 9,5 eingestellt, dann wurde mit Wasser auf 100 g aufgefüllt. Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Die Ergebnisse der Haarfärbeversuche sind der folgenden Tabelle zu entnehmen:

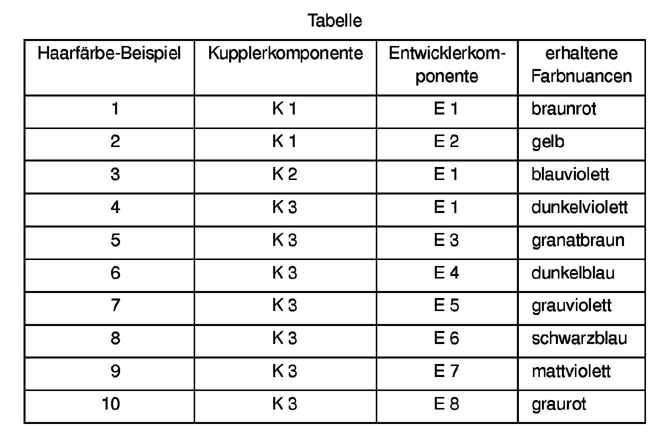

Die Haarfärbungen verfügten über hervorragende Farbintensitäten und Echtheitseigenschaften.

## Patentansprüche

1. Verwendung von Hydroxynaphthalinen der allgemeinen Formel 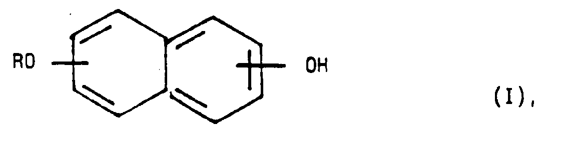 wobei R für eine C₁-C₄-Alkyl-, eine C₂-C₄-Hydroxyalkyl-, eine Phenyl- oder eine Benzylgruppe steht, zum Färben von Keratinfasern, insbesondere menschlichen Haaren.

2. Verwendung nach Anspruch 1, wobei R in der Formel für eine C₁-C₄-Alkylgruppe steht.

3. Verwendung nach Anspruch 1 und 2, wobei die Hydroxynaphthaline der Formel 1 ausgewählt sind aus 1-Hydroxy-6-methoxynaphthalin, 1-Hydroxy-7-methoxynaphthalin, 2-Hydroxy-6-methoxynaphthalin und 2-Hydroxy-7-methoxynaphthalin.

4. Verwendung von Hydroxynaphthalinen der Formel nach Anspruch 1 bis 3 als Kupplerkomponenten in Oxidationsfärbemitteln gemeinsam mit üblichen Entwicklerkomponenten.

5. Verwendung nach Anspruch 4, dadurch gekennzeichnet, daß die Entwicklerkomponenten ausgewählt sind aus p-Toluylendiamin, 2,4,5,6-Tetraaminopyrimidin, p-Aminophenol, N,N-Bis-(2-hydroxy-ethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol.

6. Oxidationsfärbemittel zum Färben von menschlichen Haaren, enthaltend Oxidationsfarbstoffvorprodukte in einem wasserhaltigen Träger, dadurch gekennzeichnet, daß mindestens ein Hydroxynaphthalin der Formel I gemäß Ansprüche 1 bis 3 als Kupplerkomponente in einer Menge von 0,05 bis 20 mMol sowie mindestens eine übliche Entwicklerkomponente in einer Menge von 0,05 bis 20 mMol und gegebenenfalls übliche direktziehende Farbstoffe in einer Menge von 0,05 bis 20 mMol, jeweils pro 100 g des Haarfärbemittels, enthalten sind.

## Claims

1. The use of hydroxynaphthalenes corresponding to general formula I: 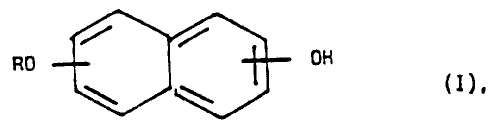 in which R is a C₁₋₄ alkyl, a C₂-₄ hydroxyalkyl, a phenyl or a benzyl group, for colouring keratin fibres, more particularly human hair.

2. The use claimed in claim 1, characterized in that R in formula I is a C₁₋₄ alkyl group.

3. The use claimed in claims 1 and 2, characterized in that the hydroxynaphthalenes corresponding to formula I are selected from 1-hydroxy-6-methoxynaphthalene, 1-hydroxy-7-methoxynaphthalene, 2-hydroxy-6-methoxynaphthalene and 2-hydroxy-7-methoxynaphthalene.

4. The use of hydroxynaphthalenes of formula I according to claims 1 to 3 as secondary intermediates in oxidation colorants together with typical primary intermediates.

5. The use claimed in claim 4, characterized in that the primary intermediates are selected from p-tolylenediamine, 2,4,5,6-tetraminopyrimidine, p-aminophenol, N,N-bis-(2-hydroxyethyl)-p-phenylenediamine, 2-(2,5-diaminophenyl)-ethanol, 2-(2,5-diaminophenoxy)-ethanol, 1-phenyl-3-carboxyamido-4-amino-5-pyrazolone, 4-amino-3-methylphenol.

6. Oxidation colorants for colouring human hair containing oxidation dye precursors in a water-containing carrier, characterized in that they contain at least one hydroxynaphthalene of formula I according to claims 1 to 3 as secondary intermediate in a quantity of 0.05 to 20 mmoles and at least one typical primary intermediate in a quantity of 0.05 to 20 mmoles and, optionally, typical substantive dyes in a quantity of 0.05 to 20 mmoles per 100 g of the hair colorant.

## Revendications

1. Utilisation d'hydroxynaphtalènes de formule générale 1 : 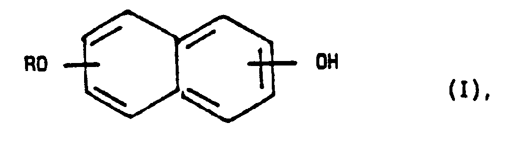 où R est un groupe alkyle en C₁₋C₄, un groupe hydroxyalkyle en C₂-C₄, un groupe phényle ou un groupe benzyle, pour teindre des fibres de kératine, notamment des cheveux humains.

2. Utilisation selon la revendication 1, où R dans la formule est un groupe alkyle en Ci-C₄.

3. Utilisation selon les revendications 1 et 2, où les hydroxynaphtalènes de formule I sont choisis parmi les 1-Hydroxy-6-méthoxynaphtalène, 1-Hydroxy-7-méthoxynaphtalène, 2-hydroxy-6-méthoxynaphtalène et 2-hydroxyméthoxy- naphtalène.

4. Utilisation d'hydroxynaphtalènes de formule I selon les revendications 1 à 3, comme composants de coupleur dans les teintures d'oxydation en présence des composants de développeur habituels.

5. Utilisation selon la revendications 4,
caractérisée en ce que
on choisit les composants de développeur parmi les : p-toluylènediamine, 2,4,5,6-tétraaminopyrimidine, p-amino- phénol, N,N-Bis-(2-hydroxyéthyl)-p-phenylènediamine, 2-(2,5-diaminophényl)-éthanol, 2-(2,5- diaminophénoxy)-éthanol, 1-phényl-3-carboxyamido-4-aminopyrazolone-5, 4-Amino-3-méthylphénol.

6. Teintures d'oxydation pour teindre les cheveux humains contenant des précurseurs de colorants d'oxydation dans un véhicule aqueux,
caractérisées en ce que
elles contiennent au moins un hydroxynaphtalène de formule 1 selon les revendications 1 à 3 comme composant de coupleur en une quantité de 0,05 à 20 mmol ainsi qu'un composant usuel de développeur en une quantité de 0,05 à 20 mmol et le cas échéant des colorants usuels montant directement sur la fibre en une quantité de 0,05 à 20 mmol, respectivement pour 100 g de teinture capillaire.
